# EUROPÄISCHE PATENTANMELDUNG

(11) **EP 2 891 488 A1**
(43) Veröffentlichungstag der Anmeldung: **08.07.2015**
(21) Anmeldenummer: 14004428.0
(22) Anmeldetag: 27.12.2014
(51) Int. Cl.: A61K 9/70

(54) **Transdermales Therapeutisches System**

(30) Priorität: 03.01.2014 DE 102014000200
(71) Anmelder: Horstmann, Michael, Dr., 56564 Neuwied (DE)
(72) Erfinder: Horstmann, Michael, Dr., 56564 Neuwied (DE)
(74) Vertreter: Flaccus, Rolf-Dieter

(57) **Zusammenfassung**

1. Mehrschichtiges, wirkstoffhaltiges Transdermales Therapeutisches System (TTS) zur Abgabe von Wirkstoffen über die Haut an den menschlichen Körper.

2.1 Bei bekannten Transdermalen Therapeutischen Systemen ist nachteilig, dass diese wegen mangelnder Kohäsion während der Applikation auf der Haut zum Verschmieren neigen und dadurch unsicher auf der Haut haften.

2.2 Zur Verbesserung der Kohäsion weist ein TTS (10) zwei oder mehrere selbstklebende Klebschichten (1, 3, 3', 3'') und mindestens eine nicht selbstklebende, zugfeste und wirkstoffdurchlässige Zwischenschicht (2, 2', 2'') auf; die genannten Klebschichten und Zwischenschicht(en) sind abwechselnd angeordnet und miteinander verbunden.

2.3 Das mehrschichtige TTS eignet sich für die Verabreichung von pharmazeutischen Wirkstoffen über die Haut zu therapeutischen Zwecken.

## Beschreibung

Die Erfindung betrifft ein mehrschichtiges wirkstoffhaltiges Pflaster (Transdermales Therapeutisches System, TTS) zur Abgabe von Wirkstoffen über die Haut an den menschlichen Körper.

Wirkstoffhaltige Pflaster sind unter der Bezeichnung transdermale therapeutische Systeme (TTS) in der arzneilichen Therapie einer Reihe von Erkrankungen bereits im Markt eingeführt. Die Vorteile dieser Form der Wirkstoffabgabe sind in erster Linie die verlängerten Applikationsintervalle, die zu einer verbesserten Patientencompliance führen, sowie die Vermeidung des first-pass-Effektes (vorzeitiger Wirkstoffabbau bei oraler Gabe) und das pharmakokinetisch optimierte Plasmakonzentrations-Zeitprofil, das eine längere Wirkdauer bei weniger Nebenwirkungen gewährleistet.

Aufgrund dieser Vorteile sind TTS seit einer Reihe von Jahren bekannt. Solche Systeme sind z. B. für Estradiol, Norethisteronacetat, Nicotin, Fentanyl, Tulobuterol, Rivastigmin, Rotigotin, Ethinylestradiol/Norelgestromin, Buprenorphin oder Nitroglycerin und eine zunehmende Reihe weiterer Wirkstoffe in die Therapie eingeführt. Transdermale Therapeutische Systeme sind in der Regel dünn und meist zweischichtig aufgebaut, so dass sich mit Hilfe der direkt der Haut zugewandten Seite (Klebschicht) eine zumindest vorübergehend klebende Verbindung zur Haut ergibt, über die der Wirkstoff (Arzneistoff) abgegeben wird.

TTS bestehen nach dem Stand der Technik üblicherweise aus einer arzneistoffundurchlässigen Trägerschicht (auch Backing oder Rückschicht genannt), einer arzneistoffhaltigen Reservoirschicht mit Kontrollmembran und Klebschicht, oder aus ein oder mehreren Matrixschichten gegebenenfalls mit einer Haftklebeschicht zur Befestigung auf der Haut, und einer vor der Applikation zu entfernenden, arzneistoffundurchlässigen Schutzschicht (sog. Release Liner).

Zur Verbesserung der Wirkstoffpermeation durch die Haut werden neben Polymeren, Harzen und anderen pharmazeutischen Hilfsstoffen auch bei Raumtemperatur flüssige Systemkomponenten eingesetzt, die unter anderem der Einstellung der Klebkraft, der Diffusionsverbesserung innerhalb des transdermalen therapeutischen Systems und/oder der Verbesserung der Wirkstoffpermeation durch die Haut dienen.

Sowohl Wirkstoffe (Arzneistoffe) wie auch insbesondere flüssige Hilfsstoffe können die bei der Herstellung störende Eigenschaft der Flüchtigkeit und/oder Thermolabilität unter Prozessbedingungen aufweisen. Um die dadurch verursachten Probleme zu vermeiden, können bei der Herstellung bestimmte, dem Fachmann bekannte Techniken zum Einsatz kommen, insbesondere kontaminationsarme Formen des Bedruckens, des Beschichtens oder Laminierens mit reinen Polymer-/Wirk-stoff-Mischungen, wie zum Bespiel nach DE 4332094 A1 oder DE 102006026060 A1.

Die Verbesserung der Kohäsion, insbesondere die Verhinderung oder Vermeidung des "kalten Flusses" von transdermalen Therapeutischen Systemen, war stets ein herausragendes Ziel der Entwicklung, da Systeme mit mangelnder Kohäsion während der Applikation auf der Haut zum Verschmieren neigen und dadurch schnell unansehnlich aussehen und unsicher haften. Diese Problematik wird durch die in der Regel weichmachende Wirkung des Wirkstoffes verstärkt.

Es gab in der Vergangenheit zahlreiche Versuche, das Problem etwa durch Zusatz von konsistenzverbessernden Stoffen bei Siliconpolymeren (EP 0524776 A1), durch Feuchtigkeitsentzug des Basispolymers, damit es für Wirkstoffe aufnahmefähiger wird (EP 2308480 A2) oder auch durch Zumischung von hydrophilen Polymeren als Zuschlagstoff zum Klebpolymer (WO 2005099676 A2) zu verbessern. Keine dieser Lösungen hat bis heute zu durchgreifend besser klebenden Systemen geführt.

Auch ist der Einsatz von häufig benötigten neu konzipierten Polymeren mittlerweile mit hohen regulatorischen Risiken verbunden, die im Extremfall das Versagen der pharmazeutischen Zulassung und damit einen Ausschluss vom Markt zur Folge haben können.

Zwar sind im Stand der Technik auch mehrschichtige transdermale therapeutische Systeme beschrieben worden (z. B. EP 2298277 A1, US 4769028 A), beispielsweise um besondere Freisetzungseigenschaften zu erzielen. Allerdings sind diese vorbekannten TTS ebenfalls mit dem Nachteil der ungenügenden Kohäsion behaftet; jedenfalls wurden keine besonderen Maßnahmen zur Verhinderung des kalten Flusses vorgesehen. Durch die ungenügende Kohäsion wird die praktische Anwendung dieser TTS erheblich erschwert.

Das Problem des kalten Flusses könnte theoretisch auch durch eine Verringerung der Kleberschichtdicke vermindert werden, da bei geringer werdender Dicke der fließfähigen Kleberanteile aufgrund des Stokesschen Gesetzes die Austrittsgeschwindigkeit am Rand dann geringer wird. Jedoch ist diese Maßnahme nur von sehr begrenztem Nutzen, da einzelne Klebschichten mit weniger als etwa 50 *µ*m Dicke auf der Haut nicht mehr gut haften, jedenfalls dann, wenn keine zusätzliche Weichmachung in der rückwärtigen Schicht vorgenommen wird.

Aufgabe der vorliegenden Erfindung ist es daher, ein transdermales therapeutisches System mit verbesserter Kohäsion bereitzustellen, bei welchem der sog. "kalte Fluss" vermieden oder zumindest verringert wird, und welches die Nachteile aus dem Stand der Technik bekannter, vergleichbarer Systeme vermeidet. Diese Aufgabe wird gelöst durch ein mehrschichtiges transdermales therapeutisches System gemäß Hauptanspruch, sowie durch die in den abhängigen Ansprüchen angegebenen Ausführungsformen.

Demzufolge bezieht sich die vorliegende Erfindung auf ein mehrschichtiges, wirkstoffhaltiges Transdermales Therapeutisches System (TTS) mit einer hautseitigen selbstklebenden Klebschicht und einer dieser Klebschicht gegenüber liegenden, wirkstoffundurchlässigen, äußeren Rückschicht. Das erfindungsgemäße TTS ist dadurch gekennzeichnet, dass es zwei oder mehrere selbstklebende Klebschichten und mindestens eine nicht selbstklebende, zugfeste und wirkstoffdurchlässige Zwischenschicht, aufweist, wobei die genannten Klebschichten und Zwischenschicht(en) abwechselnd angeordnet und miteinander verbunden sind, wodurch ein mehrschichtiges Laminat gebildet wird.

Die Eigenschaft "zugfest" bedeutet insbesondere, dass die Zugfestigkeit bei 10 % Elongation mindestens 0,1 N/mm², vorzugsweise mindestens 0,5 N/mm², besonders bevorzugt mindestens 2 N/mm² beträgt. Die Meßmethode zur Bestimmung der Zugfestigkeit wird weiter unten erläutert.

Vorzugsweise weisen die nicht selbstklebenden Zwischenschichten (Folienschichten) des TTS eine Zugfestigkeit auf, die mindestens der fünffachen, insbesondere mindestens der zehnfachen Zugfestigkeit der Klebschicht(en) entspricht (bei 10 % Elongation). Falls das jeweilige TTS zwei oder mehrere Klebschichten aufweist, die sich bezüglich ihrer Zugfestigkeit unterscheiden, beziehen sich die vorstehenden Angaben (mindestens fünffach bzw. zehnfach) auf die Klebschicht mit dem höchsten Zugfestigkeitswert.

Die Angaben zur Zugfestigkeit der nicht selbstklebenden Schichten bzw. der Klebschichten beziehen sich auf den Herstellungszustand der jeweiligen Folie oder Schicht, d. h. auf den Zustand der einzelnen Folie oder Schicht vor dem Zusammenfügen zu einem Laminat bzw. TTS.

Überraschenderweise hat sich gezeigt, dass die Zugfestigkeit des gesamten Systems (TTS) - bei gleichbleibender Gesamt-Schichtdicke - zunimmt, wenn die Anzahl der einzelnen Schichten (Kleb- und Zwischenschichten), aus denen das System aufgebaut ist, erhöht wird (wobei die einzelnen Schichten eine entsprechend geringere Schichtdicke aufweisen sollten, damit die Gesamtdicke des Systems gleichbleibt).

Im Rahmen der Herstellung des beschriebenen Systems werden nach bekannten Verfahren zunächst Einzelschichten auf bereitgestellten dehäsiv ausgerüsteten Stützfolien (Trägerfolien), in der Regel aus PET (Polyethylenterephthalat), im lösemittelhaltigen Beschichtungsverfahren mit Rakel-, Schlitzdüsen-, Sprüh- oder Rollenauftrag in gleichmäßigen Schichtdicken mit etwa 10-80 (bevorzugt 20-50) g/m² Auftragsgewicht aufgetragen und anschließend getrocknet. Die Angaben zur Schichtdicke beziehen sich auf den Zustand nach der Trocknung.

Im Falle beidseitiger abgestufter Siliconisierung kann mit dem Substrat direkt in sich gewickelt werden. Das Aufeinanderlaminieren der Systemkomponenten, d. h. der einzelnen Schichten des TTS, kann in beliebiger Reihenfolge ausgeführt werden, jedoch unter Beachtung der erfindungsgemäßen Kombination von Klebschichten und Zwischenschichten.

Der erfindungsgemäße, überraschende Erfolg ergibt sich aus der Kombination zweier oder mehrerer, durch folienförmige Zwischenschichten separierter Klebschichten, insbesondere selbstklebender Schichten, mit wirkstoffdiffusiblen (d. h. wirkstoffdurchlässigen), aber mechanische Stabilität (d. h. Kohäsion) verleihenden Zwischenschichten, die mit den jeweils benachbarten Klebschichten verbunden sind. Vorzugsweise wird jeweils zwischen zwei benachbarten Klebschichten eine Zwischenschicht angeordnet, unter Ausbildung eines Schichtverbundes.

Dabei ist die Zugabe des Wirkstoffes, insbesondere eines Arzneistoffs, in eine oder mehrere Schichten möglich, sowohl lösemittelhaltig nach Zwischentrocknungsprozessen, als auch lösemittelfrei, wenn der Wirkstoff bei Verarbeitungstemperatur flüssig ist oder einem in der Formulierung verbleibenden Lösemittel zugesetzt ist. Der/die Wirkstoff(e) bzw. Arzneistoff(e) kann in einer oder mehreren Klebschichten, und/oder in einer oder mehreren Zwischenschichten enthalten sein. Gemäß einer weiteren Ausführungsform wird der/den Zwischenschicht(en) kein Wirkstoff zugesetzt, so dass die Zwischenschicht(en) im Herstellungszustand frei von Wirkstoff(en) ist/sind.

Die Klebstoffschichten und Zwischenschichten des erfindungsgemäßen TTS können in beliebiger Sequenz hergestellt und nach dem Fachmann bekannten Prozessen aufeinander laminiert werden. Optional kann noch eine wiederablösbare Schutzschicht, welche die hautseitige Klebschicht bedeckt und vor der Verwendung des TTS entfernt wird, hinzugefügt werden.

Als Grundpolymere für die Herstellung der selbstklebenden Schicht(en) (Klebstoffschicht(en)) sind folgende bevorzugt: Styrol-Isopren-Copolymere, Polyisobutylen, Silicone, Acrylatcopolymere, Butylkautschuk, Polybutylen, Styrol-Isopren, Styrol-Copolymer, Styrol-Butadien-Styrol-Blockcopolymere. Diese Grundpolymere können sowohl für die Herstellung der hautseitigen Klebschicht als auch der weiteren selbstklebenden Klebschicht(en) verwendet werden.

Als Acrylatcopolymere kommen insbesondere Alkylacrylatcopolymere (vorzugsweise C₁-C₁₄ Alkyl), insbesondere Butylmethacrylat-Methylmethacrylat-Copolymere, in Betracht. Allgemein kommen als (Co-)Monomere Ester der Acrylsäure oder Methacrylsäure in Betracht, wie z. B. Methylacrylat, Ethylacrylat, Propylacrylat, Methylmethacrylat, Ethylmethacrylat, Butylacrylat, Butylmethacrylat, Isobornylacrylat, Isobornylmethacrylat, Benzylacrylat, Benzylmethacrylat, Laurylacrylat, Stearylacrylat, Isooctylacrylat, 2-Ethylhexylacrylat, 2-Ethylhexylmethacrylat, sowie Acrylsäure und Methacrylsäure.

Die vorstehende Aufstellung von Grundpolymeren ist nicht einschränkend gemeint; grundsätzlich eignen sich alle selbstklebenden, physiologisch verträglichen Polymere, einzeln oder in Kombination (Gemische).

Vorzugsweise besteht das zur Herstellung der selbstklebenden Klebschichten, vorzugsweise sämtlicher selbstklebender Klebschichten, verwendete Grundmaterial zu mindestens 40 Gew.-%, vorzugsweise zu mindestens 50 Gew.-%, insbesondere zu mindestens 60 Gew.%, aus den genannten Grundpolymeren, vorzugweise aus Polyisobutylen, Acrylestercopolymeren, Siliconklebemassen oder Mischungen aus zwei oder mehreren der vorstehend genannten Grundpolymere. Die Angaben in Gew.-% beziehen sich jeweils auf das Trockengewicht.

Ferner kann das erwähnte Grundmaterial einen oder mehrere Zuschlagstoffe enthalten; derartige, für haftklebende Schichten geeignete Zusatzstoffe sind dem Fachmann bekannt. Insbesondere Komponenten wie Harze (Klebharze), Öle, Füllstoffe und andere pharmazeutisch übliche Hilfsstoffe können vorteilhaft benutzt werden.

Die Dicke der Schichten ist im Allgemeinen nicht beschränkt, allerdings können sich aufgrund der verwendeten Herstellungsverfahren Einschränkungen ergeben. Beispielsweise kann die Schichtdicke nach unten hin durch die Präzision der Auftragsverfahren begrenzt sein, und nach oben hin durch die verbleibende Verfahrensökonomie der Trocknung, die mit höherer Dicke aufwendiger wird. So können Flächengewichte zwischen 10 und 150 g/m² (bevorzugt 20 bis 50 g/m²; jeweils im getrockneten Zustand) als typisch eingestuft werden.

Vorzugsweise weisen die selbstklebenden Schichten und/oder die hautseitige Klebschicht eine Klebkraft (Schälkraft 90°) auf Stahl von mindestens 1 N/cm², bevorzugt mindestens 10 N/cm² auf.

Die in einem erfindungsgemäßen TTS enthaltenen Klebschichten können entweder dieselbe Dicke oder/und Zusammensetzung aufweisen, oder sie können sich hinsichtlich Dicke oder/und Zusammensetzung unterscheiden. Auch kann der Wirkstoffgehalt in den einzelnen Klebschichten eines TTS gleich oder unterschiedlich sein.

Als Grundpolymere für die Herstellung der nicht selbstklebenden Zwischenschicht(en) sind folgende bevorzugt: Methacrylatcopolymere, hochmolekulares Polyisobutylen (vorzugsweise > 150.000 g/mol, insbesondere > 1.100.000 g/mol), Polyvinylalkohol, Polyvinylpyrrolidon, Styrol-Isopren-Copolymere, Stärkederivate, Pullulan, Cellulosederivate (z. B. Carboxymethylcellulose, Ethylcellulose, Methylcellulose, Hydroxymethylcellulose, Hydroxypropylmethylcellulose).
Als Methacrylatcopolymere kommen insbesondere auch Aminoalkylmethacrylat-Copolymere in Betracht, vorzugsweise Diethylaminoethylreste enthaltende Copolymere.

Die vorstehende Aufstellung von Grundpolymeren ist nicht einschränkend gemeint; grundsätzlich eignen sich alle nicht selbstklebenden, physiologisch verträglichen Polymere und ihre Gemische.

Bezüglich der nicht selbstklebenden Zwischenschichten wird des Weiteren bevorzugt, dass diese eine Klebkraft auf Stahl (Schälkraft 90°) von unter 0,01 N/cm², bevorzugt unter 0,001 N/cm² aufweisen.

Vorzugsweise besteht das Grundmaterial für die Herstellung der nicht selbstklebenden Zwischenschichten (2, 2', 2''), vorzugsweise sämtlicher Zwischenschichten, zu mindestens 40 Gew.-%, vorzugsweise zu mindestens 50 Gew.-%, insbesondere zu mindestens 60 Gew.%, aus den genannten Grundpolymeren, bevorzugt Polyvinylpyrrolidon, Methacrylatcopolymeren, Ethinylenvinylacetat, Polyvinylacetat, Polyethylen, modifizierter Cellulose, modifizierter Stärke, Polyvinylalkohol oder Mischungen aus zwei oder mehreren der vorstehend genannten Stoffe. Die Angaben in Gew.-% beziehen sich jeweils auf das Trockengewicht.

Im allgemeinen kann die Zugfestigkeit der nicht selbstklebenden Zwischenschichten durch eine Steigerung des Polymeranteils und/oder eine entsprechende Verringerung des Füllstoff-Anteils verbessert werden. Deshalb kann es zwecks Verbesserung der Zugfestigkeit von Vorteil sein, wenn der Polymeranteil in den Zwischenschichten auf mindestens 70 Gew.-%, vorzugsweise mindestens 80 Gew.-%, insbesondere mindestens 90 Gew.-% erhöht wird.

Ferner können die nichtklebenden Zwischenschichten einen oder mehrere Zuschlagstoffe enthalten; hierfür geeignete Zusatzstoffe sind dem Fachmann bekannt. Bei der Auswahl von Art und Menge der Zuschlagstoffe sollte darauf geachtet werden, dass die unter Verwendung dieser Zuschlagstoffe hergestellten (Zwischen-)Schichten zugfest sind, insbesondere, dass sie eine Zugfestigkeit von über 0,1 N/mm², bevorzugt von über 2 N/mm² aufweisen (bei 10 % Elongation).

Die Dicke der Zwischenschichten ist im Allgemeinen nicht beschränkt, allerdings können sich aufgrund der verwendeten Herstellungsverfahren Einschränkungen ergeben. Beispielsweise kann die Schichtdicke nach unten hin durch die Präzision der Auftragsverfahren begrenzt sein, und nach oben hin durch die verbleibende Verfahrensökonomie der Trocknung, die mit höherer Dicke aufwendiger wird. So können Flächengewichte zwischen 10 und 150 g/m² (bevorzugt 20 bis 50) als typisch eingestuft werden.

Die Zwischenschicht(en) ist/sind wirkstoffdiffusible (wirkstoffdurchlässige) Schichten oder Folien, d. h. sie sind für den/die im TTS enthaltene(n) Wirkstoff(e), insbesondere Arzneistoff(e), durchlässig. Vorzugsweise sind die Zwischenschicht(en), oder zumindest eine der Zwischenschichten, als Folie ausgebildet oder haben eine folienförmige Gestalt.

Die in einem erfindungsgemäßen TTS enthaltenen Zwischenschichten können entweder dieselbe Dicke oder/und Zusammensetzung aufweisen, oder sie können sich hinsichtlich Dicke oder/und Zusammensetzung unterscheiden. Als Zwischenschichten können auch im Handel erhältliche Folienmaterialien ausgewählt werden, sofern sie die erfindungsgemäß geforderte Zugfestigkeit (siehe oben) aufweisen.

Alle Schichten des TTS (sowohl Klebstoffschichten als auch Zwischenschichten) können durch konventionelle Techniken des Lösens, Mischens, Beschichtens und temperaturgeschützten Trocknens oder auch der nur durch Wärme geprägten Formung erzeugt werden. Bei den Prozessen der letztgenannten Art, die gemeinhin auch als Extrusion bezeichnet werden, wird die Masse durch Kneter plastifiziert und durch eine Schlitzdüse beschichtend aufgetragen.

Die für die Herstellung der erwähnten wirkstoffundurchlässigen Rückschicht (wie auch der ablösbaren Schutzschicht) geeigneten Materialien sind dem Fachmann bekannt (z. B. Folien aus PET, Polyethylen, Polypropylen, PVC; Metallfolien). Die Rückschicht bewirkt zum einen, dass ein eventueller Wirkstoffverlust nach außen verhindert wird, und zum anderen, dass das TTS vor einem eventuellen Verkleben mit Textilien (des Patienten) geschützt wird.

Der Wirkstoffanteil kann in denjenigen Schichten, in denen gemäß obiger Beschreibung ein solcher Anteil vorgesehen ist, vorzugsweise 0,1 bis 60 Gew.-%, insbesondere 1 bis 30 Gew.-% oder 5 bis 25 Gew.-% betragen. In jedem Fall ergänzen sich die Anteile des/der Grundpolymers/e, der Zuschlagstoffe (optional) und der Wirkstoffe (optional) in dem jeweiligen Grundmaterial bzw. in der jeweiligen Schicht (Klebschicht oder nicht selbstklebende Schicht) zu 100 Gew.-%, bezogen auf den getrockneten Zustand.

Der Zusatz der Wirkstoffe kann durch Zusatz in alle, einzelne oder auch nur eine einzige dieser Schichten erfolgen. Dabei können Herstellungsverfahren gewählt werden, bei denen Wirkstoff(e) und Hilfsstoff(e) (wie Grundpolymere, Zuschlagstoffe, Hilfsstoffe) gemeinsam aufgelöst, suspendiert oder dispergiert werden, mit anschließender Beschichtung und Trocknung. Es können beispielsweise auch Herstellungsverfahren gewählt werden, bei denen der Wirkstoff (insbesondere ein Arzneistoff) selbst als Lösemittel (oder Suspendier- bzw. Dispergierflüssigkeit) des Grundpolymers bzw. der Grundpolymere und/oder der Zuschlagstoffe verwendet wird.

In Einzelfällen, z. B. bei Verwendung flüchtiger Wirkstoffen, kann es vorteilhaft sein, wenn zumindest eine der vorzugsweise folienförmigen Zwischenschichten aus den gleichen Grundpolymeren (und ggf. Zuschlagstoffen) zwei- oder mehrlagig aufgebaut ist. Dabei werden die Grundpolymere und ggf. Zuschlagstoffe der folienförmigen Zwischenschicht in einem flüssigen Wirkstoff (insbesondere in einem flüchtigen Wirkstoff) gelöst und als eine zweite Lage auf eine zuvor (vorzugsweise aus demselben Grundmaterial) hergestellte erste Lage aufgetragen, bei welcher es sich um eine reine, d. h. ohne Wirkstoffzusatz hergestellte Lage handelt. Die anschließend stattfindende, schnelle Äquilibrierung des Wirkstoffes innerhalb der beiden miteinander verbundenen Lagen erzeugt eine einheitliche, wirkstoffhaltige Zwischenschicht.

In jedem Fall ist aufgrund der geringen Diffusionswege eine spontane Verteilung des Wirkstoffs in den Systemkomponenten durch Äquilibrierung, falls gewünscht innerhalb von Stunden bis Tagen nach der Herstellung, leicht möglich.

Die Gesamtdicke der erfindungsgemäßen mehrschichtigen Systeme (TTS) beträgt vorzugsweise 50 *µ*m bis 2 mm, insbesondere 100 *µ*m bis 500 *µ*m.

Die Zugfestigkeit (in N/mm²) wird auf an sich bekannte Weise mittels Zugversuch (Zugprüfung) ermittelt. Hierzu werden aus dem zu untersuchenden Grundmaterial (Folie oder Schicht), aus der zu untersuchenden Schicht des TTS, oder aus dem zu untersuchenden TTS (gesamtes System) Probenkörper (Zugproben) hergestellt, beispielsweise durch Ausstanzen. Die zur Bestimmung der Zugfestigkeit verwendeten Probenkörper sind nicht mit einer Träger- oder Stützfolie ausgestattet, da die Anwesenheit einer solchen Folie das Messergebnis verfälschen würde.

Die Probenkörper bzw. Stanzlinge weisen jeweils eine Breite von 1 cm auf, mit linear-parallel verlaufenden Kanten (in Längsrichtung). Zwecks besserer Vergleichbarkeit der Messungen sollten die Probenkörper eine in etwa gleiche Dicke aufweisen; unbedingt erforderlich ist dies jedoch nicht, da sich die Zugkraft proportional zur Probendicke verhält und eine Umrechnung deshalb leicht möglich ist.

Der Probenkörper wird zwischen die Klemmbacken einer Zugprüfmaschine eingespannt, wobei die gesamte Messlänge des Probenkörpers 5 cm beträgt. Diese Länge entspricht dem zwischen den gegenüberliegenden Klemmbacken befindlichen Abschnitt des Probenkörpers.

Sodann wird der Probenkörper in der Zugprüfmaschine mit konstanter Verformungsgeschwindigkeit (1 % Elongation pro Sekunde) gedehnt und die Längenänderung des Probenkörpers gemessen. Der Probenkörper wird dabei einer integrierenden Kraftmessung unterzogen, wobei der Mittelwert über 5 mm Elongation (= 10 % Elongation) bestimmt wird. Die Angabe der Zugdehnung in N/mm² bezieht sich auf die bei 10 % Elongation gemessenen Werte.

Die Messung erfolgt bei 25 °C, 50 % relativer Luftfeuchtigkeit und einem Luftdruck von 800 bis 1060 hPa; die Probenkörper werden vor der Messung über einen Zeitraum von mindestens 12 h unter den genannten Bedingungen gelagert (äquilibriert).

### BEISPIELE

### Beispiel 1:

### Herstellung eines erfindungsgemäßen Systems (TTS)

a) 20 g Polyisobutylen (z. B. Oppanol^{®} 100, BASF; ca. 1.110.000 g/mol) werden in 60 g n-Heptan vollständig aufgelöst und mit einer Spaltbreite von ca. 70 *µ*m auf eine siliconisierte PET-Folie (50 *µ*m Dicke), welche als Stütz- oder Trägerfolie dient, beschichtet. Nach Trocknung über 4 min bei 60 °C und anschließend 5 min bei 80 °C wird eine gleichmäßige selbstklebende Schicht von 17 g/m² erhalten.
b) 20 g neutrales Polymethylmethacrylat (z. B. Plastoid^{®} B, Evonik; Butylmethacrylat-Methylmethacrylat-Copolymer, ca. 150.000 g/mol) werden in 60 g Ethylacetat vollständig aufgelöst und mit einer Spaltbreite von ca. 120 *µ*m auf siliconisierte PET-Folie (50 *µ*m) beschichtet. Nach Trocknung über 20 min bei 70 °C wird eine gleichmäßige, nicht selbstklebende Schicht (Zwischenschicht) von 30 g/m² auf dem Substrat (PET-Folie) erhalten.
c) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie (50 *µ*m) beschichtet. Nach Trocknung über 7 min bei 60 °C und anschließend 10 min bei 80 °C wird eine gleichmäßige selbstklebende Schicht von 25 g/m² erhalten.
d) 5 g alkalisches Polymethylmethacrylat (z. B. Eudragit^{®} E 100, Evonik; Dimethylaminoethylmethacrylat-Butylmethacrylat-Methylmethacrylat-Copolymer, ca. 47.000 g/mol) werden in 30 g Miglyol^{®} (Triglyceridgemisch von Fettsäuren mittlerer Kettenlänge, im wesentlichen Capryl-, Caprin- und Laurinsäure) und 20 g Rivastigmin-Base (Arzneistoff/Wirkstoff) vollständig gelöst.
e) 1 g Polyisobutylen (z. B. Oppanol^{®} B200, BASF; ca. 4.000.000 g/mol), 10 g Polyisobutylen (z. B. Oppanol^{®} B100, BASF), 2 g Polyisobutylen (z. B. Oppanol^{®} B10, BASF; ca. 40.000 g/mol) und 5 g Paraffinöl werden in 50 g n-Heptan vollständig aufgelöst und mit einer Spaltbreite von ca. 200 *µ*m auf siliconisierte PET-Folie (100 *µ*m) beschichtet. Nach Trocknung über 20 min bei 70 °C und anschließend 15 min bei 95 °C wird eine gleichmäßige, selbstklebende Schicht von 50-52 g/m² erhalten.
f) Eine 20 *µ*m starke PET-Folie wird auf das Zwischenprodukt aus a) klebseitig laminiert und die siliconisierte PET-Folie (50 *µ*m; Stützfolie) entfernt und verworfen. Die PET-Folie (20 *µ*m) bildet die Rückschicht des Systems (TTS). Das entstandene Laminat wird klebseitig auf das Zwischenprodukt aus b) laminiert und die siliconisierte PET-Folie (50 *µ*m) entfernt und verworfen.

Die nicht selbstklebende, aus Zwischenprodukt b) stammende Seite (d. h. die nicht selbstklebende Zwischenschicht) des immer dicker gewordenen Laminates wird auf das Zwischenprodukt c) - selbstklebende Schicht von 25 g/m² - auflaminiert; die siliconisierte PET-Folie (50 *µ*m) wird entfernt und verworfen.

Die aus dem Zwischenprodukt c) stammende selbstklebende Oberfläche bzw. Klebschicht wird mit der wirkstoffhaltigen Lösung aus d) mit 25 g/m² beschichtet (unter Ausbildung einer nichtklebenden, wirkstoffhaltigen Zwischenschicht) und gleichzeitig oder anschließend klebseitig Zwischenprodukt e) auflaminiert (selbstklebende Schicht). Die siliconisierte PET-Folie (100 *µ*m) bildet die Schutzschicht (release liner) des so gebildeten mehrschichtigen TTS.

Die Reihenfolge der miteinander verbundenen Schichten dieses TTS ist wie folgt (von distal/außen nach proximal/hautseitig): Rückschicht (PET-Folie, 20 *µ*m); Klebschicht (Polyisobutylen; 17 g/m²); nichtklebende Zwischenschicht (neutrales Polymethylmethacrylat; selbstklebende Schicht (Polyisobutylen, 25 g/m²); Zwischenschicht (alkalisches Polymethylmethacrylat); selbstklebende Schicht (Polyisobutylen, 50-52 g/m²); Schutzschicht (siliconisierte PET-Folie, 100 1*µ*m).

Nach etwa 24 Stunden Äquilibrierungszeit werden Stanzlinge von 20 cm² gestanzt. Sie können unmittelbar zu therapeutischen Zwecken auf die Haut aufgeklebt werden, nachdem die Schutzschicht (siliconisierte PET-Folie, 100 *µ*m) entfernt und verworfen wurde.

### Beispiel 2:

### Herstellung eines weiteren erfindungsgemäßen Systems (TTS)

a) 14 g Polyisobutylen (z. B. Oppanol^{®} B100, BASF) und 5 g Polyisobutylen niedrigeren Molekulargewichts (z. B. Oppanol^{®} B10, BASF; ca. 40.000 g/mol) werden in 60 g n-Heptan vollständig aufgelöst und mit einer Spaltbreite von ca. 100 *µ*m auf siliconisierte PET-Folie (50 *µ*m Dicke), welche als Stütz- oder Trägerfolie dient, beschichtet. Nach Trocknung über 7 min bei 60 °C und anschließend 10 min bei 80 °C wird eine gleichmäßige selbstklebende Schicht von 25 g/m² erhalten.
b) 20 g alkalisches Polymethylmethacrylat (z. B. Eudragit^{®} E 100, Evonik; siehe oben) werden in 60 g Ethylacetat vollständig aufgelöst und mit einer Spaltbreite von ca. 120 *µ*m auf siliconisierte PET-Folie (Stützfolie, 50 *µ*m) beschichtet. Nach Trocknung über 20 min bei 70 °C wird eine gleichmäßige nicht selbstklebende Schicht von 30 g/m² auf dem Substrat erhalten.
c) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie (50 *µ*m) beschichtet. Nach Trocknung über 7 min bei 60 °C und anschließend 10 min bei 80 °C wird eine gleichmäßige selbstklebende Schicht von 25 g/m² erhalten.
d) 5 g alkalisches Polymethylmethacrylat (z. B. Eudragit^{®} E 100, Evonik; siehe oben) werden in 30 g Nicotin-Base vollständig gelöst.
e) 20 g Polyisobutylen (z. B. Oppanol^{®} 100, BASF) und 5 g Paraffinöl werden in 50 g n-Heptan vollständig aufgelöst und mit einer Spaltbreite von ca. 200 *µ*m auf siliconisierte PET-Folie (100 *µ*m) beschichtet. Nach Trocknung über 20 min bei 70 °C und anschließend 15 min bei 95 °C wird eine gleichmäßige selbstklebende Schicht von 50-52 g/m² erhalten.
f) Eine 20 *µ*m starke PET-Folie wird auf das Zwischenprodukt aus a) klebseitig laminiert und die siliconisierte PET-Folie (50 *µ*m) entfernt und verworfen.
   Das entstandene Laminat wird klebseitig auf das Zwischenprodukt aus b) laminiert und die siliconisierte PET-Folie (50 *µ*m) entfernt und verworfen.
   Die nicht selbstklebende, aus Zwischenprodukt b) stammende Seite des immer dicker gewordenen Laminates wird auf Zwischenprodukt c) auflaminiert, die siliconisierte PET-Folie (50 *µ*m) entfernt und verworfen.
   Die selbstklebende Oberfläche wird mit der Lösung aus d) mit 30 g/m² beschichtet und gleichzeitig klebseitig Zwischenprodukt e) auflaminiert.
   Nach mindestens 24 Stunden Äquilibrierungszeit werden Stanzlinge von 20 cm² gestanzt. Sie können unmittelbar zu therapeutischen Zwecken auf die Haut aufgeklebt werden, nachdem die siliconisierte PET-Folie (100 *µ*m) entfernt und verworfen wurde.

### Beispiel 3:

### Herstellung eines weiteren erfindungsgemäßen Systems

a) 14 g Polyisobutylen (z. B. Oppanol^{®} B100, BASF) und 5 g Polyisobutylen niedrigeren Molekulargewichts (z. B. Oppanol^{®} B10, BASF) werden in 60 g n-Heptan vollständig aufgelöst und mit einer Spaltbreite von ca. 100 *µ*m auf unbehandelte PET-Folie (20 *µ*m) beschichtet. Nach Trocknung über 7 min bei 60 °C und anschließend 10 min bei 80 °C wird eine gleichmäßige selbstklebende Schicht von 25 g/m² erhalten.
b) 20 g alkalisches Polymethylmethacrylat (z. B. Eudragit^{®} E 100, Evonik) wird in 60 g Ethylacetat vollständig aufgelöst und mit einer Spaltbreite von ca. 120 *µ*m auf siliconisierte PET-Folie (50 *µ*m) beschichtet. Nach Trocknung über 20 min bei 70 °C wird eine gleichmäßige nicht selbstklebende Schicht von 30 g/m² auf dem Substrat erhalten.
c) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie (100 *µ*m) beschichtet. Nach Trocknung über 7 min bei 60 °C und anschließend 10 min bei 80 °C wird eine gleichmäßige selbstklebende Schicht von 25 g/m² erhalten.
d) 5 g alkalisches Polymethylmethacrylat (z. B. Eudragit^{®} E 100, Evonik) werden in 30 g Nicotin-Base vollständig gelöst.
e) Das Zwischenprodukt aus a) wird auf das Zwischenprodukt aus b) klebseitig laminiert und die siliconisierte PET-Folie (50 *µ*m), der Hilfsträger aus b), entfernt und verworfen.
   Auf die offene, nicht selbstklebende PolymethylmethacrylatSeite des entstandenen Laminates wird die Lösung aus d) mit
   20 g/m² beschichtet und anschließend mit seiner Klebseite Zwischenprodukt c) auflaminiert.
f) Nach mindestens 24 Stunden Äquilibrierungszeit werden Stanzlinge von 20 cm² gestanzt. Sie können unmittelbar zu therapeutischen Zwecken auf die Haut aufgeklebt werden, nachdem die siliconisierte PET-Folie (100 *µ*m) entfernt und verworfen wurde.

### Beispiel 4:

### Herstellung eines weiteren erfindungsgemäßen Systems

a) 14 g Polyisobutylen (z. B. Oppanol^{®} B100, BASF) und 5 g Polyisobutylen niedrigeren Molekulargewichts (z. B. Oppanol^{®} B10, BASF) werden in 60 g n-Heptan vollständig aufgelöst und mit einer Spaltbreite von ca. 100 *µ*m auf siliconisierte (schwächer trennende Ausführung) PET-Folie (100 *µ*m) beschichtet. Nach Trocknung über 7 min bei 60 °C und anschließend 10 min bei 80 °C wird eine gleichmäßige selbstklebende Schicht von 25 g/m² erhalten.
b) 20 g alkalisches Polymethylmethacrylat (z. B. Eudragit^{®} E 100, Evonik) werden in 60 g Ethylacetat vollständig aufgelöst und mit einer Spaltbreite von ca. 120 *µ*m auf siliconisierte PET-Folie (50 *µ*m) beschichtet. Nach Trocknung über 20 min bei 70 °C wird eine gleichmäßige nicht selbstklebende Schicht von 30 g/m² auf dem Substrat erhalten.
c) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie (50 *µ*m) beschichtet. Nach Trocknung über 7 min bei 60 °C und anschließend 10 min bei 80 °C wird eine gleichmäßige selbstklebende Schicht von 25 g/m² erhalten.
d) Das Zwischenprodukt aus a) wird klebseitig auf das Zwischenprodukt aus b) laminiert und die siliconisierte PET-Folie (50 *µ*m) entfernt und verworfen. Das Zwischenprodukt wird klebseitig auf die Klebseite von Zwischenprodukt aus c) laminiert und nach Entfernung der siliconisierten PET-Folie (50 *µ*m) offen bereitgehalten.
e) 14 g Polyisobutylen (z. B. Oppanol^{®} B100, BASF) und 5 g Polyisobutylen niedrigeren Molekulargewichts (z. B. Oppanol^{®} B10, BASF) werden in 60 g n-Heptan vollständig aufgelöst und mit einer Spaltbreite von ca. 100 *µ*m auf unbehandelte PET-Folie (21 *µ*m) beschichtet. Nach Trocknung über 7 min bei 60 °C und anschließend 10 min bei 80 °C wird eine gleichmäßige selbstklebende Schicht von 25 g/m² erhalten.
f) 20 g alkalisches Polymethylmethacrylat (z. B. Eudragit^{®} E 100, Evonik) werden in 60 g Ethylacetat vollständig aufgelöst und mit einer Spaltbreite von ca. 120 *µ*m auf siliconisierte PET-Folie (50 *µ*m) beschichtet. Nach Trocknung über 20 min bei 70 °C wird eine gleichmäßige nicht selbstklebende Schicht von 30 g/m² auf dem Substrat erhalten.
g) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie (50 *µ*m) beschichtet. Nach Trocknung über 7 min bei 60 °C und anschließend 10 min bei 80 °C wird eine gleichmäßige selbstklebende Schicht von 25 g/m² erhalten.
h) Das Zwischenprodukt aus e) wird klebseitig auf das Zwischenprodukt aus f) laminiert und die siliconisierte PET-Folie (50 *µ*m) entfernt und verworfen. Das Zwischenprodukt wird klebseitig auf die Klebseite von Zwischenprodukt aus g) laminiert und nach Entfernung der siliconisierten PET-Folie (50 *µ*m) offen bereitgehalten.
i) 5 g alkalisches Polymethylmethacrylat (z. B. Eudragit^{®} E 100, Evonik) werden in 30 g Nicotin-Base vollständig gelöst.
j) Die Lösung aus i) wird mit 25 g/m² auf die offenliegende selbstklebende Oberfläche aus d) beschichtet und anschließend klebseitig Zwischenprodukt h) auflaminiert.
   Nach mindestens 24 Stunden Äquilibrierungszeit werden Stanzlinge von 20 cm² gestanzt. Sie können unmittelbar zu therapeutischen Zwecken auf die Haut aufgeklebt werden, nachdem die siliconisierte PET-Folie (100 *µ*m) entfernt und verworfen wurde.

### Beispiel 5:

### Herstellung eines weiteren erfindungsgemäßen Systems

a) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie (50 *µ*m) beschichtet. Nach Trocknung über 7 min bei 60 °C und anschließend 10 min bei 80 °C wird eine gleichmäßige selbstklebende Schicht von 25 g/m² erhalten.
b) 20 g Polyvinylpyrrolidon (z.B. Kollidon^{®} 90) und 30 g Rotigotin werden in 120 g Ethanol vollständig aufgelöst und mit einer Spaltbreite von ca. 120 *µ*m auf siliconisierte PET-Folie 50 *µ*m beschichtet. Nach Trocknung über 10 min bei 70 °C wird eine gleichmäßige nicht selbstklebende Schicht von 22 g/m² auf dem Substrat erhalten.
c) 20 g Polyisobutylen (z. B. Oppanol^{®} 100, BASF) werden in 60 g n-Heptan vollständig aufgelöst und mit einer Spaltbreite von ca. 100 *µ*m auf siliconisierte PET-Folie 50 *µ*m beschichtet. Nach Trocknung über 7 min bei 60 °C und anschließend 10 min bei 80 °C wird eine gleichmäßige selbstklebende Schicht von 25 g/m² erhalten.
d) 20 g Polyvinylpyrrolidon (z.B. Kollidon^{®} 90) und 30 g Rotigotin werden in 120 g Ethanol vollständig aufgelöst und mit einer Spaltbreite von ca. 120 *µ*m auf siliconisierte PET-Folie (50 *µ*m) beschichtet. Nach Trocknung über 10 min bei 70 °C wird eine gleichmäßige nicht selbstklebende Schicht von 22 g/m² auf dem Substrat erhalten.
e) 20 g Polyisobutylen (z. B. Oppanol^{®} 100, BASF) und 5 g Paraffinöl werden in 50 g n-Heptan vollständig aufgelöst und mit einer Spaltbreite von ca. 200 *µ*m auf siliconisierte PET-Folie (100 *µ*m) beschichtet. Nach Trocknung über 20 min bei 70 °C und anschließend 15 min bei 95 °C wird eine gleichmäßige selbstklebende Schicht von 50-52 g/m² erhalten.
f) Eine 20 *µ*m starke PET-Folie wird auf das Zwischenprodukt aus a) klebseitig laminiert und die siliconisierte PET-Folie 50 *µ*m entfernt und verworfen.

Das entstandene Laminat wird klebseitig auf das Zwischenprodukt aus b) laminiert und die siliconisierte PET-Folie 50 *µ*m entfernt und verworfen.

Die nicht selbstklebende, aus Zwischenprodukt b) stammende Seite des immer dicker gewordenen Laminates wird auf Zwischenprodukt c) auflaminiert, die siliconisierte PET-Folie 50 *µ*m entfernt und verworfen.
Das entstandene Laminat wird klebseitig auf das Zwischenprodukt aus d) laminiert und die siliconisierte PET-Folie (50 *µ*m) entfernt und verworfen. Auf die Oberfläche wird anschließend klebseitig Zwischenprodukt e) auflaminiert.

Nach etwa 1 Stunde Äquilibrierungszeit werden Stanzlinge von 30 cm² gestanzt. Sie können unmittelbar zu therapeutischen Zwecken auf die Haut aufgeklebt werden, nachdem die siliconisierte PET-Folie (100 *µ*m) entfernt und verworfen wurde.

### Beispiel 6:

### a) Herstellung eines erfindungsgemäßen Teil-Laminats

Aus einer Lösung (30 Gew.-%) von Plastoid^{®} B in Ethylacetat wird eine nichtklebende Schicht mit einer Schichtdicke von 10 *µ*m hergestellt. Plastoid^{®} B (Evonik Industries AG) ist ein Copolymer aus Butylmethacrylat und Methylmethacrylat mit einer durchschnittlichen gewichtsmittleren Molmasse von 150.000 g/mol.

Des Weiteren wird eine selbstklebende Schicht gemäß obigem Beispiel 5e) hergestellt, mit einer Schichtdicke von 20 *µ*m. Ober- und Unterseite dieser selbstklebenden Schicht werden jeweils mit einer nicht selbstklebenden Schicht aus Plastoid^{®} B (siehe oben) bedeckt und zu einem dreischichtigen Laminat zusammengefügt. Anschließend werden durch Laminieren noch fünf weitere Schichten (nicht selbstklebende bzw. Klebschichten) mit dem dreischichtigen Laminat zusammengefügt. Die Schichtreihenfolge ist derartig, dass auf eine nicht selbstklebende (Zwischen-)Schicht jeweils eine Klebschicht folgt und umgekehrt.

Aus dem so erhaltenen Laminat mit einer Gesamt-Schichtdicke von ca. 50 *µ*m werden durch Stanzen Probenkörper für Zugfestigkeitsmessungen gewonnen, wie weiter oben beschrieben.

### b) Herstellung eines Vergleichsbeispiels

Als Vergleichsbeispiel dient ein dreischichtiges Laminat, welches wie folgt hergestellt wird:
Aus einer Lösung (30 Gew.-%) von Plastoid^{®} B in Ethylacetat wird eine nicht selbstklebende Schicht mit einer Schichtdicke von 25 *µ*m hergestellt. Des Weiteren wird eine selbstklebende Schicht gemäß Beispiel 5e) hergestellt, mit einer Schichtdicke von 20 *µ*m. Ober- und Unterseite dieser selbstklebenden Schicht werden jeweils mit einer nicht selbstklebenden Schicht aus Plastoid^{®} B (25 *µ*m; siehe oben) bedeckt und zu einem dreischichtigen Laminat zusammengefügt.

Aus dem so erhaltenen dreischichtigen Laminat mit einer Gesamt-Schichtdicke von ca. 50 *µ*m werden durch Stanzen Probenkörper für Zugfestigkeitsmessungen gewonnen, wie weiter oben beschrieben.

### c) Zugfestigkeits-Messungen

Die aus dem erfindungsgemäßen Teil-Laminat bzw. dem dreischichtigen Vergleichs-Laminat hergestellten Probenstücke wurden einer Zugfestigkeitsprüfung unterzogen (wie weiter oben im Detail beschrieben). Bei annähernd gleicher Dicke des Gesamtlaminats wurden mit den erfindungsgemäßen Teil-Laminaten Zugfestigkeitswerte erreicht, die durchschnittlich doppelt so hoch waren wie die Zugfestigkeitswerte des Vergleichsbeispiels.

### ZEICHNUNGEN

Die beigefügten Zeichnungen dienen lediglich der Veranschaulichung der Erfindung, ohne dass eine Einschränkung des Schutzbereichs beabsichtigt ist. Die dargestellten Formen und Größenverhältnisse sind nicht maßstabsgetreu.

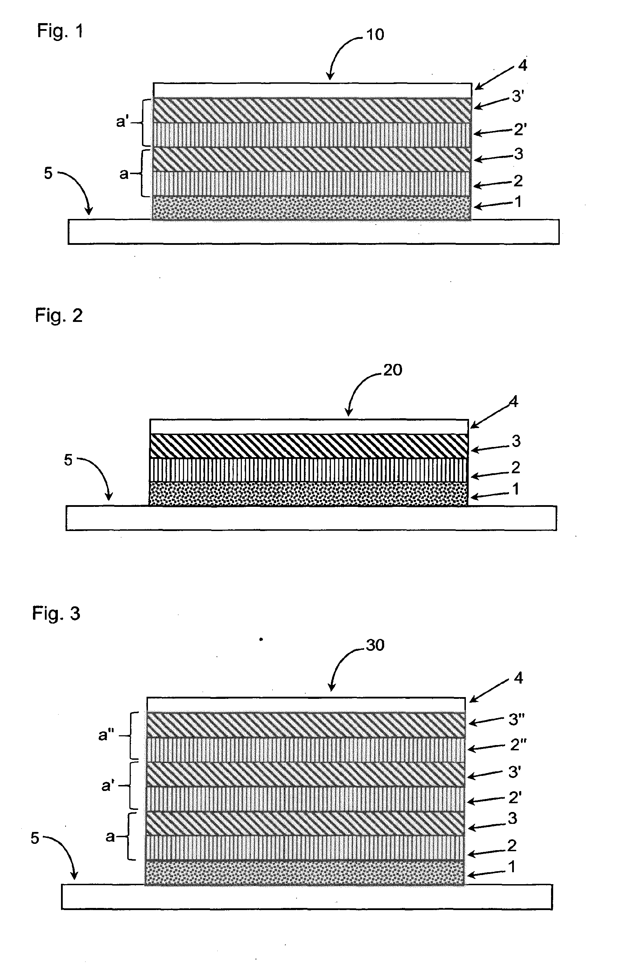

Figur 1 zeigt - in Schnittdarstellung - eine Ausführungsform eines erfindungsgemäßen mehrlagigen Transdermalen Therapeutischen Systems (10) nach Anspruch 1. Bei der hier gezeigten Ausführungsform ist eine wiederholt angeordnete Doppelschicht (a, a''), jeweils aus einer selbstklebenden Schicht (3, 3') und einer nicht selbstklebenden Zwischenschicht (2, 2'), vorhanden.
   (1) hautseitige Klebschicht (selbstklebend)
   (2), (2') nicht selbstklebende, zugfeste und wirkstoffdiffusible Schicht
   (3), (3') (weitere) selbstklebende Schicht (Klebschicht) (a), (a') einmal wiederholt angeordnete Doppelschicht, jeweils bestehend aus einer nicht selbstklebenden, zugfesten und wirkstoffdiffusiblen (d. h. wirkstoffdurchlässigen) Zwischenschicht (2) bzw. (2') und einer selbstklebenden Schicht (3) bzw. (3').
   (4) wirkstoffundurchlässige Rückschicht (außen / distal)
   (5) Trennfolie (Release Liner), auf hautseitiger Klebschicht (1).
Figur 2 zeigt eine weitere Ausführungsform eines erfindungsgemäßen mehrlagigen Transdermalen Therapeutischen Systems (20) nach Anspruch 1, jedoch ohne Wiederholung einer Doppelschicht.
   (1) selbstklebende hautseitige Schicht
   (2) nicht selbstklebende, zugfeste und wirkstoffdiffusible Schicht (folienförmige Zwischenschicht)
   (3) (weitere) selbstklebende Schicht
   (4) wirkstoffundurchlässige Rückschicht.
   (5) Trennfolie (Release Liner), auf hautseitiger Klebschicht (1).
Figur 3 zeigt eine weitere Ausführungsform eines erfindungsgemäßen mehrlagigen Transdermalen Therapeutischen Systems nach Anspruch 1, mit zweifacher Wiederholung einer aus Klebschicht und Zwischenschicht bestehenden Doppelschicht.
   (1) selbstklebende hautseitige Schicht
   (2) nicht selbstklebende, zugfeste und wirkstoffdiffusible Schicht (Zwischenschicht, folienförmig)
   (3) (weitere) selbstklebende Schicht (Klebschicht)
   (2), (2'), (2'') nicht selbstklebende, zugfeste und wirkstoff-diffusible Schicht (Folie)
   (3), (3'), (3'') (weitere) selbstklebende Schicht (Klebschicht)
   (a), (a'), (a'') : zweimal wiederholt angeordnete Doppelschicht, jeweils bestehend aus einer nicht selbstklebenden, zugfesten und wirkstoffdiffusiblen Zwischenschicht (2, 2', 2'') und einer selbstklebenden Schicht (3, 3', 3''). Insgesamt sind drei Doppelschichten (a, a', a'') benachbart angeordnet und miteinander verbunden.
   (4) wirkstoffundurchlässige Rückschicht (außen / distal)
   (5) Trennfolie (Release Liner), auf hautseitiger Klebschicht (1).

## Patentansprüche

1. Mehrschichtiges, wirkstoffhaltiges Transdermales Therapeutisches System, mit einer hautseitigen selbstklebenden Klebschicht (1) und einer dieser Klebschicht gegenüber liegenden, wirkstoffundurchlässigen, äußeren Rückschicht (4), **dadurch gekennzeichnet, dass** es zwei oder mehrere selbstklebende Klebschichten (1, 3, 3', 3'') und mindestens eine nicht selbstklebende, zugfeste und wirkstoffdurchlässige Zwischenschicht (2, 2', 2'') aufweist, wobei die genannten Klebschichten und Zwischenschicht(en) abwechselnd angeordnet und miteinander verbunden sind.

2. Transdermales Therapeutisches System nach Anspruch 1, **dadurch gekennzeichnet, dass** die Zwischenschicht(en) (2, 2', 2''), oder zumindest eine der Zwischenschichten, als Folie ausgebildet ist/sind.

3. Transdermales Therapeutisches System nach Anspruch 1 oder 2, **dadurch gekennzeichnet, dass** es mindestens eine Doppelschicht (a, a', a'') aufweist, welche aus einer nicht selbstklebenden, zugfesten und wirkstoffdiffusiblen Zwischenschicht (2, 2', 2'') und einer damit verbundenen, selbstklebenden Schicht (3, 3', 3'') besteht.

4. Transdermales Therapeutisches System nach Anspruch 3, **dadurch gekennzeichnet, dass** es mindestens zwei, vorzugsweise zwei oder drei der genannten Doppelschichten (a, a', a'') in aufeinanderfolgender Anordnung enthält, wobei jeweils zwei benachbarte Doppelschichten mittels einer selbstklebenden Schicht (3, 3', 3'') miteinander verbunden sind.

5. Transdermales Therapeutisches System nach einem der vorangehenden Ansprüche, **dadurch gekennzeichnet, dass** die selbstklebenden Klebschichten (1, 3, 3', 3''), vorzugsweise sämtliche selbstklebende Klebschichten (1, 3, 3', 3''), zu mindestens 40 Gew.-%, vorzugsweise zu mindestens 50 Gew.-%, insbesondere zu mindestens 60 Gew.%, aus Polyisobutylen, Acrylestercopolymeren, Siliconklebemassen oder Mischungen aus zwei oder mehreren der vorstehend genannten Stoffe bestehen.

6. Transdermales Therapeutisches System nach einem der vorangehenden Ansprüche, **dadurch gekennzeichnet, dass** die nicht selbstklebenden Zwischenschichten (2, 2', 2''), vorzugsweise sämtliche Zwischenschichten, zu mindestens 40 Gew.-% , vorzugsweise zu mindestens 50 Gew.-%, insbesondere zu mindestens 60 Gew.%, aus Polyvinylpyrrolidon, Methacry-latcopolymeren, Ethinylenvinylacetat, Polyvinylacetat, Polyethylen, modifizierter Cellulose, modifizierter Stärke, Polyvinylalkohol oder Mischungen aus zwei oder mehreren der vorstehend genannten Stoffe bestehen.

7. Transdermales Therapeutisches System nach einem der vorangehenden Ansprüche, **dadurch gekennzeichnet, dass** die Zugfestigkeit der Zwischenschicht(en) (2, 2', 2'') mindestens 0,1 N/m², insbesondere mindestens 0,5 N/mm², besonders bevorzugt mindestens 2 N/mm² beträgt.

8. Transdermales Therapeutisches System nach einem der vorangehenden Ansprüche, **dadurch gekennzeichnet, dass** die Zugfestigkeit der Zwischenschicht(en) (2, 2', 2'') mindestens der fünffachen, insbesondere mindestens der zehnfachen Zugfestigkeit der Klebschicht(en) entspricht (bei 10 % Elongation).

9. Transdermales Therapeutisches System nach einem der vorangehenden Ansprüche, **dadurch gekennzeichnet, dass** die Zwischenschicht(en) (2, 2', 2'') eine Klebkraft auf Stahl (Schälkraft 90°) von unter 0,01 N/cm², bevorzugt unter 0,001 N/cm² aufweist/aufweisen.

10. Transdermales Therapeutisches System nach einem der vorangehenden Ansprüche, **dadurch gekennzeichnet, dass** die selbstklebenden Schichten (3, 3', 3'') sowie die hautseitige Klebschicht (Schicht 1) eine Klebkraft (Schälkraft 90°) auf Stahl von mindestens 1 N/cm², bevorzugt mindestens 10 N/cm² aufweisen

11. Transdermales Therapeutisches System nach einem der vorangehenden Ansprüche, **dadurch gekennzeichnet, dass** die hautseitige Klebschicht (1) zum Schutz ihrer Oberfläche vor Anwendung durch eine ablösbare Trennfolie (Release Liner) geschützt ist.

12. Transdermales Therapeutisches System nach einem der vorangehenden Ansprüche, **dadurch gekennzeichnet, dass** zumindest eine der Zwischenschichten (2, 2', 2''), vorzugsweise sämtliche Zwischenschichten, Polyvinylpyrrolidon enthalten, wobei dessen Anteil vorzugsweise mindestens 10 Gew.-%, insbesondere mindestens 20 Gew.-%, besonders bevorzugt mindestens 30 Gew.-% beträgt.

13. Transdermales Therapeutisches System nach einem der vorangehenden Ansprüche, **dadurch gekennzeichnet, dass** die Klebschichten (3, 3', 3''), vorzugsweise sämtliche der Klebschichten (3, 3', 3''), zum überwiegenden Teil (d. h. mindestens 50 Gew.-%, insbesondere mindestens 60 Gew.-%) aus Polyisobutylen, Acrylsäurecopolymeren oder Siliconpolymeren bestehen.

14. Transdermales Therapeutisches System nach einem der vorangehenden Ansprüche, **dadurch gekennzeichnet, dass** die Klebschichten (3, 3', 3'') zum überwiegenden Teil (d. h. mindestens 50 Gew.-%, insbesondere mindestens 60 Gew.-%) aus Polyisobutylen oder Siliconpolymeren bestehen, und dass die Zwischenschichten (2, 2', 2'') Polyvinylpyrrolidon sowie einen oder mehrere Wirkstoffe enthalten, deren Löslichkeit in Polyvinylpyrrolidon mindestens doppelt so hoch ist wie die Löslichkeit in einem 1:1 (w/w)-Gemisch von Polyvinylpyrrolidon und Wasser.

15. Transdermales Therapeutisches System nach einem der vorangehenden Ansprüche, **dadurch gekennzeichnet, dass** als Wirkstoff Rotigotin enthalten ist.

16. Transdermales Therapeutisches System nach einem der vorangehenden Ansprüche, **dadurch gekennzeichnet, dass** es aus einer selbstklebenden hautseitigen Schicht (1), zwei übereinander angeordneten Doppelschichten (a, a') aus jeweils einer zugfesten, wirkstoffdiffusiblen Zwischenschicht (2, 2') und einer selbstklebenden Schicht (3, 3'), sowie einer im wesentlichen wirkstoffundurchlässigen Rückschicht (4) besteht, und wobei bevorzugt wird, dass die selbstklebenden Schichten (3, 3') zum überwiegenden Teil (d. h. mindestens 50 Gew.-%, insbesondere mindestens 60 Gew.-%) aus Polyisobutylen bestehen und die Zwischenschichten (2, 2') Methacrylat-Polymere enthalten.

17. Transdermales Therapeutisches System nach einem der vorangehenden Ansprüche, **dadurch gekennzeichnet, dass** es aus einer selbstklebenden hautseitigen Schicht (1), drei übereinander angeordneten Doppelschichten (a, a', a'') aus jeweils einer zugfesten, wirkstoffdiffusiblen Zwischenschicht (2, 2', 2'') und einer selbstklebenden Schicht (3, 3', 3''), sowie einer im wesentlichen wirkstoffundurchlässigen Rückschicht (4) besteht, und wobei bevorzugt wird, dass die selbstklebenden Schichten (3, 3', 3'') zum überwiegenden Teil (d. h. mindestens 50 Gew.-%, insbesondere mindestens 60 Gew.-%) aus Polyisobutylen bestehen und die Zwischenschichten (2, 2', 2'') Methacrylat-Polymere enthalten.

18. Transdermales Therapeutisches System nach einem der vorangehenden Ansprüche, **dadurch gekennzeichnet, dass** als Wirkstoff(e) ein weichmachender, lipöphiler und bei 20°C zu mindestens 0,1% (w/w) in Ethanol löslicher Wirkstoff, oder ein Gemisch solcher Wirkstoffe, enthalten ist.

19. Transdermales Therapeutisches System nach einem der vorangehenden Ansprüche, **dadurch gekennzeichnet, dass** als Wirkstoff Nicotin-Base enthalten ist.
